Europäisches Patentamt

**European Patent Office**

Office européen des brevets

(11) Numéro de publication: **0 531 191 B1**

(12) FASCICULE DE BREVET EUROPEEN

(45) Date de publication de fascicule du brevet: **14.06.95**   (51) Int. Cl.6: **C07C 15/08**, C07C 7/00

(21) Numéro de dépôt: **92402355.9**

(22) Date de dépôt: **27.08.92**

(54) **Procédé et dispositif de séparation du paraxylène dans des hydrocarbures aromatiques avec une adsorption en lit mobile simulé et une cristallisation.**

(30) Priorité: **05.09.91 FR 9111004**
**06.07.92 FR 9208497**
**06.07.92 FR 9208498**

(43) Date de publication de la demande:
**10.03.93 Bulletin 93/10**

(45) Mention de la délivrance du brevet:
**14.06.95 Bulletin 95/24**

(84) Etats contractants désignés:
**BE DE DK ES IT NL**

(56) Documents cités:
**EP-A- 0 003 622**
**DE-A- 2 436 076**
**GB-A- 1 420 796**
**US-A- 3 813 452**
**US-A- 4 118 429**

**PATENT ABSTRACTS OF JAPAN vol. 5, no. 12 (C-40)(684) 24 Janvier 1981**

(73) Titulaire: **INSTITUT FRANCAIS DU PETROLE**
**4, Avenue de Bois Préau**
**F-92502 Rueil-Malmaison (FR)**

(72) Inventeur: **Hotier, Gérard**
**39, avenue Berthelot**
**F-92500 Rueil Malmaison (FR)**
Inventeur: **Roux Guerraz, Claude**
**52, Rue Claude Decaen**
**F-75011 Paris (FR)**
Inventeur: **Nguyen Thanh, Than**
**22 bis, rue de Saint Fargeau**
**F-75020 Paris (FR)**

Rank Xerox (UK) Business Services
(3.10/3.09/3.3.3)

**Description**

La présente invention décrit un procédé et un dispositif, améliorés de séparation des hydrocarbures aromatiques en $C_8$ à partir d'une charge les contenant en majorité soit tous : o-xylène, m-xylène, p-xylène, éthylbenzène, soit au moins deux d'entre eux ; l'application est l'obtention de p-xylène à un degré de pureté suffisant pour la synthèse par exemple de l'acide téréphtalique.

Une méthode connue de séparation du p-xylène consiste à procéder à une cristallisation fractionnée. On peut citer, parmi les procédés actuellement exploités, les procédés Chevron, Krupp, Amoco, Mazuren et Arco (brevets US 3,177,255 ; US 3,467,724).

- Les procédés Amoco et Arco utilisent la procédure suivante : la charge, contenant au moins 20% de p-xylène est refroidie de - 50°C à - 70°C, ce qui provoque la cristallisation. On sépare, par filtration, d'une part un gâteau de cristaux dont la teneur globale en paraxylène est de 85 à 90%, ledit gâteau étant imbibé de liquide, et d'autre part, la liqueur mère, contenant encore 7 à 8% de p-xylène. Le gâteau de cristaux est refondu et à nouveau refroidi vers - 10°C ce qui provoque une recristallisation ; après une filtration on obtient un nouveau gâteau humide et une seconde liqueur mère contenant de 25% à 40% environ de p-xylène. Le gâteau est lavé avec du toluène, ce qui permet d'obtenir une pureté finale d'au moins 99,5% après élimination du toluène par distillation. Les liqueurs mères peuvent subir un traitement d'isomérisation pour la première ou être recyclées à la charge en ce qui concerne la seconde.

- L'autre méthode de séparation des hydrocarbures aromatiques en $C_8$ et plus particulièrement du p-xylène des trois autres est une méthode de chromatographie liquide dite à contre-courant simulé (US 2 985 589) qui utilise la propriété de certains adsorbants, en particulier des zéolites, d'adsorber sélectivement le p-xylène. Les procédés Parex et Aromax utilisent cette méthode. On peut citer aussi la méthode de chromatographie liquide à co-courant simulé décrite dans le brevet US 4,402,832.

La méthode de séparation du p-xylène par cristallisation a pour principal inconvénient le fait que le taux de récupération maximal par passe est limité à environ 60% par suite de l'existence d'eutectiques entre le p-xylène et les autres hydrocarbures aromatiques en $C_8$ et il en résulte la nécessité d'une boucle d'isomérisation importante. De plus, le refroidissement vers -65°C entraîne une dépense énergétique considérable.

- Les procédés par chromatographie liquide continue (par exemple contre-courant simulé) présentent les caractéristiques suivantes : si l'on veut obtenir simultanément une pureté élevée du p-xylène (environ 99,5%) et un taux élevé de récupération (par exemple 92%), on est obligé de diviser les colonnes d'adsorbant en un grand nombre de lits (en général 24 lits) et de imiter la productivité de l'unité ; il est démontré que pour une charge contenant environ 20% de p-xylène et 15% d'éthylbenzène, si l'on veut obtenir une pureté de p-xylène de 99,5% et un taux de récupération de 92%, on ne peut dépasser une production de 0,04 $m^3$ de p-xylène par mètre cube d'adsorbant et par heure. Le principal inconvénient de ces procédés réside dans l'investissement élevé dû à la complexité de l'unité. Un autre inconvénient réside dans le fait qu'un ratio solvant/charge élevé est nécessaire pour obtenir une grande pureté (au minimum 2,1 $m^3/m^3$) soit au moins 10 $m^3$ de solvant par $m^3$ de paraxylène produit. Il en résulte des dépenses énergétiques importantes pour séparer par distillation le solvant de l'extrait et du raffinat.

Un procédé a été proposé (US 3,939,221 de British Petroleum Chemical) qui combine une première étape de cristallisation sur une charge débarrassée par distillation d'une grande part de l'o-xylène et une deuxième étape de séparation par chromatographie liquide à contre-courant simulé pour épuiser en paraxylène la liqueur mère issue de l'étape de cristallisation. Ce procédé est une simple juxtaposition des deux procédés existants, cristallisation en deux étapes (-65°C et -15°C) et chromatographie liquide continue sur une charge ne contenant que 7 à 8% de paraxylène. Il ne conduit ni à une simplification ni à une modification fondamentale d'aucune des deux étapes, il nécessite un investissement double et son seul avantage est de réduire la taille de la boucle d'isomérisation, ce qui est obtenu aussi bien par le procédé de chromatographie en contre-courant simulé pris seul.

L'art antérieur est par ailleurs illustré par les brevets suivants :
- Le brevet EP-A-003622 décrit un procédé de préparation et de séparation de paraxylène avec une étape d'adsorption sur un silicate contenant du fer et éventuellement du gallium et de l'aluminium permettant d'obtenir un premier mélange contenant du paraxylène et de l'éthylbenzène dans une proportion pondérale 1 : 1 et un second mélange contenant de l'orthoxylène et du métaxylène. La purification de ce premier mélange est réalisée entre -15°C et -80°C et plus spécifiquement à -60°C selon l'exemple.

2

- Le brevet GB 1 420 796 décrit un procédé de séparation en phase vapeur de C$_8$ aromatiques dans au moins deux zones en parallèle. L'adsorbat contient du paraxylène et de l'éthylbenzène en proportion sensiblement identique et en outre renferme 9% de métaxylène. Cet adsorbat est ensuite soumis à une cristallisation.

- Le brevet US 3,813,452 décrit un procédé de séparation d'un mélange contenant des C$_8$ aromatiques et des C$_8$ non aromatiques qui délivre dans- une zone de fractionnement, en tête une fraction non aromatique contenant 5% de paraxylène. Cette fraction de tête est soumise à une cristallisation (-40 °C - 70 °C) et le paraxylène est récupéré. Par contre, la fraction de queue riche en C$_8$ aromatiques est séparée dans une zone d'adsorption, éventuellement à contre-courant simulé et le paraxylène est récupéré. De plus, l'adsorption et la cristallisation ne sont pas couplés.

- Le brevet JP-A-55-139 327 décrit une adsorption d'un mélange d'aromatiques en C$_8$ suivie d'une cristallisation du paraxylène obtenu, entre + 10 °C et - 20 °C. Cependant l'adsorption est réalisée dans un pseudo lit mobile à trois zones. Il en résulte que la circulation continue du liquide ne peut être réalisée. D'autre part le taux de solvant sur charge atteint des valeurs très élevées (5 : 1 dans l'exemple). Par ailleurs, il est suggéré un procédé de cristallisation avec un lavage au paraxylène haute pureté ou à l'eau. Il en résulte alors une teneur en paraxylène dans le filtrat d'au moins 60% et donc un rendement en paraxylène extrait plus faible. Dans ces conditions, le filtrat ne peut être réintroduit dans la zone d'adsorption qu'en un endroit différent de celui de la charge. Celle-ci ne contient en effet que 17 à 22% de paraxylène.

L'objet de l'invention est de remédier aux inconvénients mentionnés. De manière générale l'invention concerne un procédé de séparation et de récupération de paraxylène contenu dans une charge d'hydrocarbures comprenant essentiellement des hydrocarbures aromatiques en C$_8$ caractérisé en ce qu'il comprend les étapes suivantes :

a) On met en contact en continu dans au moins une zone d'adsorption à lit mobile simulé, ladite charge contenant du métaxylène, du paraxylène, de l'éthylbenzène et éventuellement de l'orthoxylène avec un lit d'adsorbant zéolitique en présence d'un solvant de désorption approprié, dans des conditions d'adsorption telles qu'on obtient une première fraction contenant du solvant, du métaxylène, de l'éthylbenzène et éventuellement de l'orthoxylène et une deuxième fraction contenant du solvant et essentiellement du paraxylène avec une pureté comprise entre 75 et 98% ;

b) On distille la première fraction pour séparer le solvant d'une part et le mélange métaxylène, éthylbenzène et éventuellement orthoxylène d'autre part ;

c) On isomérise ledit mélange dans des conditions appropriées en présence d'hydrogène dans une zone d'isomérisation et l'on récupère un isomérat que l'on recycle vers l'étape a) ;

d) on distille la deuxième fraction et l'on récupère le solvant d'une part et le paraxylène avec une pureté de 75 à 98% d'autre part ;

e) On procède à une cristallisation du paraxylène de l'étape d) dans une zone de cristallisation à une température comprise entre + 10 °C et - 25 °C et l'on obtient d'une part une liqueur mère que l'on recycle vers l'étape a) et d'autre part des cristaux de paraxylène imbibés de liqueur mère ; et

f) On lave avec un solvant de lavage approprié les cristaux de paraxylène dans une zone de lavage et on récupère les cristaux de paraxylène à un très grand degré de pureté, soit généralement au moins 99,3% et de préférence au moins 99,7%.

L'invention consiste donc à couper de manière originale et simplifiée les deux procédés (la chromatographie liquide avec circulation continue du liquide et la cristallisation pour créer des conditions plus économiques de production du paraxylène de grande pureté.

Le lit mobile simulé peut être un lit à contre-courant simulé ou un lit à co-courant simulé.

Selon une caractéristique du procédé à lit mobile simulé, les conditions opératoires et le choix de l'adsorbant sont choisis de telle façon que la première fraction corresponde à un raffinat (composé le moins sélectivement adsorbé) et que la deuxième fraction corresponde à un extrait (composé le plus sélectivement adsorbé).

Selon une autre caractéristique du procédé à lit mobile simulé, les conditions opératoires et le choix de l'adsorbant sont choisis de telle façon que la première fraction soit un extrait et que la deuxième fraction soit un raffinat.

Dans le procédé selon l'invention, la charge est tout d'abord traitée par chromatographie liquide, selon un procédé décrit dans le brevet US 2,985,589 à contre-courant simulé ou selon un procédé décrit dans le brevet US 4,402,832, à co-courant simulé, contenant au moins 4 zones. Cette technique est utilisée pour produire selon l'invention un paraxylène de faible pureté (75 à 98%, de préférence 85 à 90%), ce qui permet une productivité considérablement améliorée (volume de charge traitée par volume d'adsorbant et par heure), un ratio solvant sur charge baissé d'au moins un tiers, un taux de récupération du paraxylène d'au moins 98% et l'emploi d'un nombre de lits distincts beaucoup plus faible : par exemple, on peut réduire ce nombre de vingt quatre à huit. L'augmentation du taux de récupération du paraxylène signifie une diminution de la taille de la boucle d'isomérisation tant par rapport au procédé de cristallisation que par rapport au procédé de chromatrographie liquide continue. On évite ainsi de forts taux de recyclage vers l'isomérisation et de ce fait un surdimensionnement de la boucle isomérisation séparation.

Selon l'invention, dans la seconde étape du procédé, on purifie la seconde fraction de la première étape (paraxylène déjà concentré). Cette étape correspond à la phase finale de purification des procédés de séparation par cristallisation existant sur le marché (par exemple le procédé ARCO). Pour une seconde fraction contenant 85 à 90% de paraxylène cette cristallisation sera avantageusement opérée entre + 5°C et -15°C. On évite ainsi les coûts opératoires prohibitifs liés à la première étape de cristallisation, ce qui permet de continuer à opérer des unités de cristallisation devenues peu rentables ou techniquement dépassées.

La liqueur mère issue de la cristallisation peut alors être recyclée à la première étape, c'est-à-dire à l'alimentation de la chromatographie en lit mobile simulé (à contre-courant ou à co-courant). D'autre part le solvant de lavage du gâteau de cristaux de paraxylène peut être redistillé.

Parmi les solvants de lavage des cristaux, on peut utiliser par exemple le n-pentane, l'eau, du paraxylène purifié ou du toluène.

Selon un mode particulièrement avantageux de mise en oeuvre, on peut également créer une synergie entre les deux étapes (chromatographie-cristallisation) si le solvant d'élution utilisé en chromatographie et le solvant de lavage du gâteau de cristaux de p-xylène employé en cristallisation est commun : alors une seule colonne de distillation du solvant suffit. Cette synergie apparaît clairement lorsque l'on utilise préférentiellement le toluène comme solvant commun aux deux étapes. Ce solvant a été décrit comme solvant d'élution pour la séparation des xylènes avec des adsorbants constitués principalement de zéolites X ou Y dont les sites échangeables sont occupés par des cations alcalins ou alcalino-terreux ; la zéolite Y échangée à la fois au baryum (45 à 65% des sites) et au potassium (35 à 55%) en particulier, donne de bons résultats. Par ailleurs, dans le procédé de cristallisation ARCO, on peut employer le toluène comme solvant de lavage du gâteau cristallin.

La juxtaposition des deux étapes impliquerait un distillation complète de la première et de la deuxième fraction de l'étape de chromatographie en lit mobile simulé (à contre-courant ou à co-courant) et une redistillation du toluène de lavage du p-xylène de la seconde étape de cristallisation. Par contre, l'intégration des deux étapes ci-dessus du procédé permet de simplifier les opérations. Du fait que l'on ne demande à la première étape qu'une performance réduite en ce qui concerne la pureté du p-xylène, il n'y a généralement pas d'inconvénient à utiliser un solvant impur comme solvant de désorption ; cette impureté peut être du métaxylène contenu dans le toluène de lavage des cristaux de la seconde étape. La liqueur mère de cristallisation peut également contenir un peu de toluène, ce qui n'est pas gênant lorsque cette dernière est recyclée à la première étape de chromatographie continue. La colonne de distillation de la deuxième fraction contenant le paraxylène de la première étape peut être réglée avec un taux de reflux tel que le toluène recueilli en tête contienne jusqu'à 2% du paraxylène impur et avec un taux de rebouillage tel que le paraxylène impur contienne jusqu'à 3% de toluène. La colonne à distiller de la première fraction contenant du métaxylène et de l'éthylbenzène de la première étape peut également être réglée avec un taux de reflux tel que le toluène contienne jusqu'à 2% de $C_8$ aromatiques. Par contre, il est préférable d'éviter de laisser du toluène dans le mélange métaxylène, éthylbenzène, orthoxylène renvoyé à l'isomérisation de manière à ne pas augmenter la taille de cette dernière. La réduction des taux de reflux de ces colonnes permet notablement de minimiser la taille des colonnes et donc de réduire les coûts énergétiques liés à la séparation du solvant. Par ailleurs, la consommation en solvant pour l'ensemble du procédé est moindre.

Généralement, la charge est une coupe d'hydrocarbures de point d'ébullition compris entre 136°C et 145°C. Lorsque la charge contient de l'orthoxylène celui-ci peut être distillé dans des conditions adéquates avant l'étape a).

Selon une caractéristique du procédé, le solvant de désorption et le solvant de lavage des cristaux peuvent être un solvant de point d'ébullition inférieur à celui de la charge tel que le toluène, ou supérieur à celui de la charge tel que le cumène. Comme il a été dit ci-avant, on préfère utiliser le toluène parce qu'il est moins cher et parce qu'il est largement utilisé dans les procédés de cristallisation existants.

Dans ce cas, le solvant récupéré lors de l'étape b) peut être recyclé dans la zone d'adsorption et/ou dans la zone de lavage, le solvant impur résultant du lavage peut être recyclé à l'étape b) et/ou à l'étape d) de distillation et le solvant résultant de l'étape d) de distillation peut être recyclé vers la zone d'adsorption et/ou vers la zone de lavage.

Selon une autre caractéristique, le solvant de désorption peut être un solvant de point d'ébullition supérieur à celui de la charge tel que le paradiéthylbenzène et le solvant de lavage peut être un solvant de point d'ébullition inférieur à celui de la charge tel que le toluène.

Le solvant récupéré lors des étapes b) et d) peut alors être recyclé dans la zone d'adsorption et le solvant impur résultant du lavage est soumis à une distillation séparée adaptée à délivrer du solvant pur recyclé vers l'étape de lavage dans la zone de cristallisation et un mélange de paraxylène, de métaxylène, d'éthylbenzène et éventuellement d'orthoxylène recyclé vers l'étape d'adsorption.

L'invention concerne aussi le dispositif et plus particulièrement une unité de séparation et de purification du paraxylène contenu dans une charge d'hydrocarbures comprenant essentiellement les hydrocarbures aromatiques en $C_8$. Cette unité comporte en combinaison :

a) une unité (8) d'adsorption dite à lit mobile simulé (à contre-courant ou à co-courant) comprenant une pluralité de colonnes (6, 7) remplies d'un adsorbant zéolitique, un moyen d'alimentation (4) en une charge, un moyen d'alimentation (11) en un solvant de désorption recyclé, un moyen (10) d'évacuation de la première fraction et un moyen d'évacuation (9) de la deuxième fraction, ladite unité d'adsorption étant adaptée à délivrer la deuxième fraction contenant le paraxylène à une pureté appropriée et avec une productivité améliorée ;

b) une première unité de distillation (12) connectée audit moyen d'évacuation de la première fraction comprenant une sortie (14) en tête et une sortie (15) en fond. Selon que le solvant est plus léger ou plus lourd que la charge celui-ci sortira en tête ou en fond de ladite unité et sera recyclé au moins en partie par le moyen (11) vers l'unité d'adsorption tandis que la première fraction débarrassée du solvant sera évacuée par ladite sortie (15) ;

c) une unité d'isomérisation (21) ayant une entrée connectée à la sortie (15) de l'unité de distillation (12) et une sortie délivrant un isomérat relié à une troisième unité de distillation (23) définie ci-dessous ;

d) une troisième unité (23) de distillation adaptée à délivrer par une sortie un isomérat distillé qui est recyclé par les moyens appropriés (2) vers l'unité d'adsorption et par une autre sortie des produits légers produits au cours de l'isomérisation ; et

e) une deuxième unité de distillation (16) connectée audit moyen d'évacuation de la deuxième fraction contenant le paraxylène (9) comprenant, soit en tête soit en fond selon que le solvant est plus léger ou plus lourd que la charge, une sortie (17) adaptée à délivrer le solvant de désorption qui est recyclé au moins en partie par le moyen de recyclage (11) vers l'unité d'adsorption et une deuxième sortie (19) adaptée à délivrer du paraxylène de pureté comprise habituellement entre 75 et 98% ;

f) au moins une unité de cristallisation du paraxylène de l'étape e) connectée à la sortie (19) adaptée à fonctionner à une température de -25°C à + 10°C, ladite unité de cristallisation comprenant en outre une unité de lavage des cristaux obtenus qui comporte une alimentation (18) en un solvant de lavage approprié, une première sortie délivrant une solution mère qui est recyclée par des moyens de recyclage (3) vers l'unité d'adsorption et une seconde sortie (25) de récupération des cristaux purs.

Selon une caractéristique du dispositif, l'unité de lavage peut comprendre une ligne de récupération de solvant impur habituellement connectée à un organe de distillation adapté à séparer du solvant pur qui est généralement recyclé vers l'unité de lavage de l'unité de cristallisation par une ligne de recyclage.

Selon une autre caractéristique du dispositif, lorsque le solvant recyclé vers l'unité d'adsorption par les moyens de recyclage et le solvant recyclé vers l'unité de lavage sont un seul et même solvant, ce dernier provient en général au moins en partie de l'unité de distillation de la deuxième fraction et/ou au moins en partie de l'unité de distillation de la première fraction qui peut aussi distiller le solvant impur comme mentionné ci-dessus, ce qui évite un surcoût en investissement.

Parmi l'ensemble des solvants susceptibles d'être utilisés, on a remarqué que les hydrocarbures monoaromatiques tels que ceux décrits dans le brevet US 4,940,830 permettaient d'obtenir de bons résultats.

Selon une autre caractéristique du dispositif, le solvant de désorption recyclé vers l'unité d'adsorption peut être le paradiéthylbenzène et le solvant recyclé vers l'étape de lavage peut être le toluène. Dans ces conditions la solution récupérée en fond de l'organe de distillation du solvant impur est recyclé vers l'unité d'adsorption par des moyens de recyclage appropriés.

Les conditions opératoires de l'unité de séparation et de récupération selon l'invention sont généralement les suivantes :

- Unité d'adsorption en contre-courant simulé : la longueur utile totale des colonnes d'adsorption est habituellement comprise entre 10 et 30 mètres et de préférence entre 15 et 25 mètres. Cette longueur est divisée en un nombre de lits compris entre 6 et 24 et de préférence de 8 à 12. Le nombre d'enceintes renfermant ces lits est compris entre 1 et le nombre desdits lits et de préférence entre 2 et 4. En outre le nombre de zones (longueur utile de colonne entre une entrée et une sortie ou vice-versa) est d'au moins 4. La température est en général comprise entre 140°C et 185°C et de préférence entre 150°C et 175°C.

La vitesse linéaire moyenne rapportée à l'enceinte du réacteur vide est comprise entre 0,4 et 1,2 cm/s et de manière préférée entre 0,8 et 1 cm/s. Le taux de solvant (rapport du débit de solvant au débit de charge) est compris entre 1,20 et 2,5 et de manière préférée entre 1,35 et 1,7.

Le taux de recyclage (rapport du débit de recyclage moyen au débit de charge) est compris entre 5 et 12 et de manière préférée entre 6 et 10.

Pour obtenir le paraxylène en tant qu'extrait, lorsque le solvant de désorption est le toluène, la zéolite préférée est une zéolite Y telle que définie dans le brevet US 3,558,730, en particulier un échange à la fois au baryum (45 à 65% des sites ) et potassium (35 à 55% des sites) donne de bons résultats. Mais, lorsque le solvant de désorption est le paradiéthylbenzène, la zéolite préférée est une zéolite X telle que définie dans le brevet US 3,558,730. En particulier un échange quasi total au baryum avec un taux résiduel en sodium inférieur à 0,3% des sites donne de bons résultats.

Dans les deux cas, la zéolite sera avantageusement employée sous forme de billes de granulométrie comprise entre 0,25 et 1 mm de diamètre et de manière préférée comprise entre 0,315 et 0,8 mm de diamètre.

Dans les deux cas, la teneur en eau de la zéolite sera maintenue en dessous de 6% poids et de manière préférée en dessous de 3% poids.

Parmi les zéolites X, celle échangée avec Ba par exemple permet aussi une bonne séparation en tant qu'extrait du paraxylène et de l'éthylbenzène comme impureté majeure. Parmi les zéolites Y adsorbant sélectivement le paraxylène, celles échangées avec un seul cation tel que K, Rb, Cs, Ag (US 4,044,062) ou les zéolites HP (high pressure) avec du lithium (US 4,615,994) sont les plus couramment utilisées pour leur bonne sélectivité. Les zéolites Y et X échangées avec deux cations K + Ba, K + Be, K + Mg, K + Rb, K + Cs, Rb + Ba, Cs + Ba, K + Cu donnent de bons résultats. On peut par ailleurs utiliser des zéolites telles que ZSM5, ZSM11 ou $\beta$ pour adsorber sélectivement le paraxylène. Dans ce cas l'éthylbenzène sera encore l'impureté majeure.

Par contre, les zéolites Y échangées par exemple avec Li, Na, Be, Mg, Ca, Sr, Mn, Cd, Cu, Ni ou avec une paire d'ions telle que Cu + Cd, Cu + Ag et Zn + Ag permettent d'adsorber sélectivement le méta- et l'orthoxylène et d'obtenir un raffinat contenant le paraxylène en majeure partie (US 4,044,062).

- Unité d'adsorption à co-courant simulé : Les conditions opératoires sont sensiblement celles que l'on utilise pour le contre-courant simulé sauf que :
- la colonne est divisée en un nombre de lits compris entre 6 et 24, de préférence 6 à 12,
- le taux de recyclage est compris entre 0,8 et 7, et de préférence entre 4 et 5,5. Le choix de l'adsorbant et du solvant est le même que celui décrit ci-avant pour l'obtention du paraxylène en tant qu'extrait ou en tant que raffinat. La mise en oeuvre en co-courant simulé permet d'obtenir un extrait de faible pureté compatible avec le procédé selon l'invention.
- Unité de cristallisation du paraxylène basse pureté. De nombreux procédés étant disponibles, on ne donnera que, par exemple, les conditions opératoires du procédé ARCO : pour une charge contenant de 85 à 90% de paraxylène, température du cristalliseur :
+5°C à - 15°C
pureté du paraxylène : 99,75%
rendement en paraxylène : 88 à 94%
teneur en paraxylène de la liqueur mère : 25 à 45%
taux de lavage : 0,8 à 2 volumes de toluène par volume de cristaux de paraxylène teneur en toluène du paraxylène purifié avant distillation finale : 80 à 98% poids.
- Unité d'isomérisation de la première fraction constituée d'un mélange de métaxylène, éthylbenzène et éventuellement orthoxylène.
De nombreux procédés sont disponibles. Certains convertissent l'éthylbenzène en xylènes tels que les procédés UOP et Engelhardt, ces procédés emploient un catalyseur bifonctionnel à base de platine sur alumine et de mordenite sous forme H tandis qu'un autre craque l'éthylbenzène en benzène et éthylène : il s'agit du procédé Mobil qui emploie un catalyseur à base de zéolite ZSM5. On donne, à titre indicatif, les conditions opératoires de la première classe de procédés :
température : 380 à 420°C
pression : 10 à 40 bar d'hydrogène
vitesse spatiale : 2 à 4 kg par kg et par heure
taux de conversion en $C_8$ aromatiques : 92 à 96%
taux de conversion de l'éthylbenzène en xylènes : 35 à 55%.

L'invention sera mieux comprise au vu du schéma illustrant de manière non limitative le procédé et le dispositif.

6

EP 0 531 191 B1

Les conditions opératoires et l'adsorption sont choisie de façon que la première fraction contenant le métaxylène et l'éthylbenzène soit un raffinat et la deuxième fraction contenant essentiellement le paraxylène soit un extrait.

On véhicule par un ligne 1 une charge comprenant environ 20% d'éthylbenzène, 18% de paraxylène, 45% de métaxylène et 17% d'orthoxylène. On y joint par une ligne 2 un effluent recyclé dont la teneur en éthylbenzène est sensiblement plus faible, typiquement 8 à 13% et qui contient des impuretés non aromatiques. Par une ligne 3 on introduit un autre effluent recyclé dont la teneur en paraxylène est plus forte, typiquement 25 à 45%. Une ligne 4 récupère la charge et ces deux effluents, elle véhicule un mélange de composition approximative, paraxylène 20 à 22,5, éthylbenzène 9 à 14%, orthoxylène 20 à 22,5%, métaxylène 45 à 50% qui est introduit dans une unité 8 de chromatographie d'adsorption en contre-courant simulé comprenant un nombre limité de colonnes 6 et 7 remplies d'un adsorbant zéolitique, chacune des colonnes étant divisée en un nombre limité de sections (le nombre total des sections de colonnes étant compris entre 8 et 12, la productivité exprimée par rapport au paraxylène produit étant d'environ 0,08 $m^3$ par $m^3$ de tamis et par heure exprimée aux conditions ambiantes. On désorbe par du toluène, à raison d'environ 1,45 $m^3$ de toluène par $m^3$ de charge, la température opératoire étant à peu près 160°C. On soutire de cette unité par une ligne 10 un raffinat appauvri en paraxylène contenant essentiellement du toluène, du métaxylène, de l'éthylbenzène et de l'orthoxylène et par une ligne 9 un extrait de composition enrichie en paraxylène contenant essentiellement du toluène et du paraxylène, l'impureté majeure étant l'éthylbenzène. Le raffinat est introduit dans une colonne à distiller 12 (température de tête 125°C, température de fond 160°C par exemple) dans laquelle est également recyclé du toluène impur par une ligne 13 provenant de l'unité de lavage d'une unité de cristallisation précisée ci-dessous. On soutire en tête par une ligne 14 du toluène (environ 37% de la quantité introduite par exemple) contenant par exemple, moins de 200 ppm de coupe $C_8$ aromatique et l'on soutire en fond de cette colonne par une ligne 15 un liquide (raffinat débarrassé du solvant) riche en éthylbenzène, en métaxylène et en orthoxylène et appauvri en paraxylène (moins de 0,5% par exemple) que l'on envoie dans une unité d'isomérisation 21. Ce raffinat est mis en contact avec de l'hydrogène introduit par une ligne 20 et avec un catalyseur à base de mordénite et de platine sur alumine à environ 380°C. Une ligne 22 conduit l'isomérat de la sortie du réacteur vers une colonne à distiller 23 (température de tête 90°C, température de fond 160°C par exemple). On soutire en tête des hydrocarbures de $C_1$ à $C_5$, de l'hexane, du cyclohexane, du benzène et du toluène et en fond de cette colonne par une ligne 2, un effluent contenant 8 à 13% d'éthylbenzène, 21 à 24% de paraxylène, 21 à 24% d'orthoxylène, de 45 à 50% de métaxylène et des impuretés non aromatiques, qui est recyclé vers l'unité de chromatographie liquide d'adsorption.

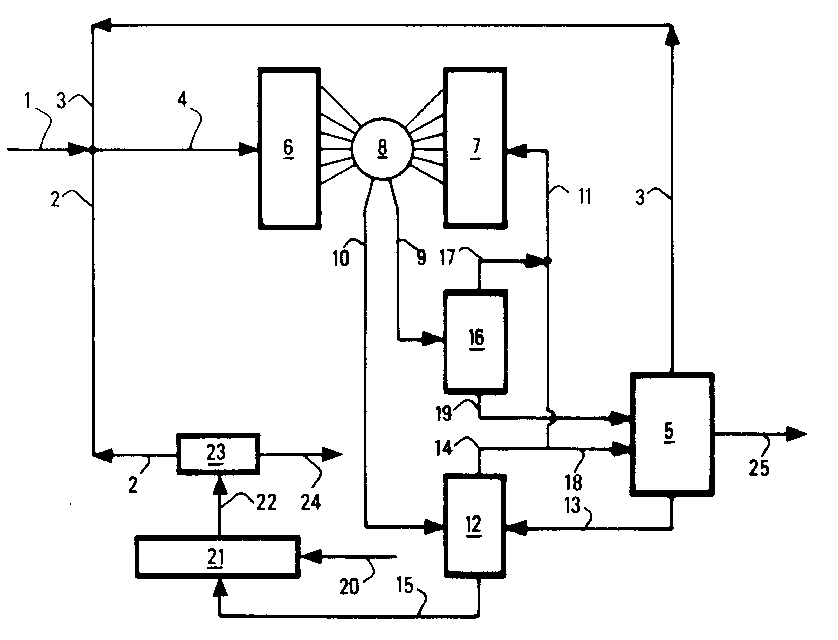

La ligne 9 introduit l'extrait dans une colonne à distiller 16 d'où l'on soutire en tête du toluène à moins de 0,2% de coupe $C_8$ aromatique (environ 63% de la quantité introduire par exemple) qui est recyclé par la ligne 11 vers l'alimentation en solvant de désorption de l'unité d'adsorption et vers l'unité de cristallisation. En fond de colonne 16 à environ 160°C on soutire le paraxylène basse pureté (à environ 90% de paraxylène) au moyen d'une ligne 19 qui le conduit dans une unité de cristallisation 5 fonctionnant à environ -10°C. Dans cette unité 5 on produit d'une part une solution ou liqueur mère appauvrie en paraxylène (environ 38%) qui est recyclée par la ligne 3 à l'entrée de l'unité de chromatographie liquide au point d'introduction de la charge et d'autre part un gâteau de cristaux de paraxylène imbibé de solution mère. Ce gâteau est centrifugé et lavé par du toluène dans une unité non représentée sur la figure. Le toluène de lavage est amené par la ligne 18 et peut provenir comme représenté sur la figure de l'unité de distillation du raffinat 12 et/ou encore de l'unité de distillation de l'extrait 16. On récupère de l'unité 5 du paraxylène refondu de pureté 99,75% par une ligne 25 et du toluène impur que l'on envoie par la ligne 13 vers la distillation 12.

On a donc décrit un mode de réalisation où le solvant de désorption de l'unité d'adsorption et le solvant de lavage de l'unité de cristallisation sont un seul et même solvant : le toluène.

Dans le cas où le paradiéthylbenzène est le solvant de désorption et le toluène le solvant de lavage, les unités de distillation 12 et 16 n'alimentent en solvant que l'unité d'adsorption. Un organe de distillation supplémentaire est alors nécessaire pour distiller le toluène utilisé dans l'unité de lavage de l'unité de cristallisation. Ce toluène sensiblement pur est ensuite recyclé vers l'unité de lavage tandis que la solution récupérée en fond de distillation est jointe à la liqueur mère et recyclée vers l'unité d'adsorption par la ligne 3.

**EXEMPLE 1.**

Cet exemple illustre un aspect particulier de l'invention : la simplification de l'unité de chromatographie liquide continue. On a réalisé une unité pilote de chromatographie liquide continue à partir de 24 colonnes en série de 1 m de longueur et de 1 cm de diamètre, la circulation entre la 24ème et la première colonne se faisant au moyen d'une pompe de recyclage. A chaque liaison intercolonne on peut injecter soit une charge à séparer soit du solvant. on peut également soutirer soit un raffinat soit un extrait. Cette unité est décrite dans un ouvrage intitulé "Preparative and production scale chromatrography processes with applications", édité par G. Barker, G. Ganestos (université de Birmingham U.K.), chapitre " From batch elution to simulated counter current chromatography" par B. Balannec et G. Hotier, (Publication de Marcel Dekker Inc, New York 1992).

L'adsorbant est constitué de zéolithe Y échangée par du potassium et par du baryum, le taux d'échange exprimé en normalité est d'environ 50% pour chacun des deux cations. La zéolithe est mise en oeuvre sous forme de billes de 0,315 à 0,5 mm de diamètre. L'ensemble des colonnes et du vannage de distribution est placé dans une étuve à 150°C.

Suivant le principe de la chromatographie à contre-courant simulé, on avance de trois colonnes toutes les six minutes à co-courant de la circulation de liquide, l'injection de solvant, le prélèvement d'extrait, l'injection de la charge et le prélèvement de raffinat.

Selon l'invention, le nombre de lits à considérer n'est donc plus que de huit. Six colonnes (donc deux lits) sont comprises entre l'injection de solvant et le prélèvement d'extrait, neuf colonnes (trois lits) sont comprises entre le prélèvement d'extrait et l'injection de charge, trois colonnes (un lit) sont comprises entre l'injection de charge et le prélèvement de raffinat et les six dernières colonnes (deux lits) se situent entre le prélèvement de raffinat et l'injection de solvant. On injecte en continu (exprimé aux conditions ambiantes) 7,2 cm³/min de toluène et 5 cm³/min de charge comprenant 21% poids de paraxylène, 17% poids d'éthylbenzène, 44% poids de métaxylène et 18% poids d'orthoxylène. On prélève également en continu 5,40 cm³/min d'extrait et 6,74 cm³/min de raffinat ; on constate environ 5‰ de pertes. Pendant la première période du cycle qui en compte huit, le solvant est injecté en colonne 1, l'extrait est prélevé à la sortie de la colonne 6, la charge est injectée en colonne 15, le raffinat est prélevé à la sortie de la colonne 18. Pendant les deux premières périodes du cycle, la pompe de recyclage débite (à température ambiante) 38,7 cm³/min, pendant la troisième période elle débite à 45,5 cm³/min, pendant les trois périodes suivantes elle débite à 40,5 cm³/min et pendant les deux dernières périodes elle débite 45,9 cm³/min. On a donc un débit de recyclage moyen de 42 cm³/min, soit un taux de recyclage moyen de 8,4 exprimé par rapport à la charge. Le paraxylène est obtenu avec une pureté de 92,2% et un taux de récupération de 98,1%. La température est de 150°C, la pression décroît à peu près linéairement de 30 bar à 5 bar. Le tableau suivant donne le bilan en régime stationnaire de l'unité :

|  | Charge | Solvant | Extrait | Raffinat |
|---|---|---|---|---|
| Débit | 5 cm³/min | 7,2 cm³/min | 5,40 cm³/min | 6,74 cm³/min |
| Toluène | - | 99,9% | 79,30% | 43,29% |
| Ethylbenzène | 17% | - | 1,07% | 11,72% |
| M Xylène | 44% | - | 0,40% | 32,30% |
| O Xylène | 18% | - | 0,15% | 12,40% |
| P xylène | 21% | - | 19,08% | 0,29% |

L'unité est limitée à une perte de charge totale de 25 bar atteinte dans ces conditions de débit. Dans ces conditions la productivité est de 0,034 m³ de paraxylène par m³ de tamis et par heure. Dans une unité industrielle, la pureté visée ne serait que de 85% par exemple et la perte de charge résultant des liaisons intercolonne serait très réduite par rapport à l'unité pilote ci-dessus. On pourrait donc augmenter la productivité en paraxylène jusqu'à 0,082 m³ par m³ et par heure en multipliant tous les débits par 1,5 et en réduisant la période de permutation en conséquence (de 3,50 minutes à 4 minutes environ au lieu de 6 minutes) et enfin en utilisant une longueur utile totale de 15 m au lieu de 24 m.

La chromatographie liquide d'adsorption à contre-courant simulé dite simplifiée selon l'invention est caractérisée par une productivité élevée par rapport à celle qui est réalisée industriellement selon l'art antérieur (environ 20% de plus dans l'exemple pilote présenté et environ 200% de plus dans une unité industrielle réalisée selon l'invention). Elle est également caractérisée par un taux de récupération du paraxylène plus élevé et une pureté nettement plus faible, ces résultats étant dus à un taux de solvant presque deux fois plus faible et un nombre de points d'injection et soutirage c'est-à-dire de lits trois fois moins importants.

**EXEMPLE 2**

Cette exemple illustre un aspect particulier de l'invention : le paraxylène est produit sous forme de raffinat. Dans l'unité pilote de l'exemple No. 1 l'adsorbant est constitué par une zéolite Y échangée par du strontium, le taux résiduel de sodium exprimé en normalité est inférieur à 3,5%. Comme précédemment la zéolite est mise en oeuvre sous forme de billes de 0,315 à 0,5 mm de diamètre.

Comme dans le cas de l'exemple No. 1, on avance de trois colonnes toutes les six minutes à co-courant de la circulation de liquide l'injection de solvant, le prélèvement d'extrait, l'injection de charge et le prélèvement de raffinat.

Selon l'invention le nombre de lits à considérer n'est donc plus que de huit. Six colonnes (donc deux lits) sont comprises entre l'injection de solvant et le prélèvement d'extrait, six colonnes sont également comprises entre le prélèvement d'extrait et l'injection de charge, six autres colonnes sont disposées entre l'injection de charge et le prélèvement de raffinat et les six dernières colonnes sont situées entre le prélèvement de raffinat et l'injection de solvant. On injecte en continu (exprimé aux conditions ambiantes) 6,8 cm$^3$/min de toluène et 4 cm$^3$/min de la charge de l'exemple 1. On prélève également en continu 9,15 cm$^3$/min d'extrait et 1,60 cm$^3$/min de raffinat (soit environ 5‰ de pertes). Pendant la première période du cycle qui en compte huit, le solvant est injecté en colonne 1, l'extrait est prélevé à la sortie de la colonne 6, la charge est injectée en colonne 12, le raffinat est prélevé à la sortie de la colonne 18. Pendant les deux premières périodes du cycle la pompe de recyclage débite à la température ambiante 40,5 cm$^3$/min, les deux périodes suivantes à 42,4 cm$^3$/min, pendant les 5ème et 6ème périodes elles débite à 38,4 cm$^3$/min, enfin pendant les deux dernières périodes du cycle elle débite à 47,3 cm$^3$/min. On a donc un débit de recyclage moyen de 42,1 cm3/min, soit un taux de recyclage de 10,52 exprimé par rapport à la charge. Le paraxylène est obtenu avec une pureté de 90,8% et un taux de récupération de 96,85%, la température est de 150°C. Le tableau suivant donne le bilan en régime stationnaire de l'unité :

|  | Charge | Solvant | Extrait | Raffinat | Pertes |
|---|---|---|---|---|---|
| Débit | 4 cm$^3$/min | 6,8 cm$^3$/min | 9,15 cm$^3$/min | 1,6 cm$^3$/min | 0,05 cm$^3$/min |
| Toluène | - | 99,9% | 66,27% | 44,00% | |
| Ethylbenzène | 17% | - | 7,08% | 1,84% | |
| M-Xylène | 44% | - | 18,86% | 1,71% | |
| O-Xylène | 18% | - | 7,54% | 1,61% | |
| P-Xylène | 21% | - | 0,25% | 50,84% | |

La pression au refoulement de la pompe de recyclage est de 30 bar, elle est de 5 bar à l'aspiration de cette même pompe.

**EXEMPLE 3**

Cet exemple illustre un aspect particulier de l'invention : la mise en oeuvre en co-courant simulé. L'unité pilote et l'adsorbant décrits dans l'exemple No. 1 sont à nouveau utilisés.

Suivant le principe de la chromatographie à co-courant simulé, on recule de quatre colonnes toutes les huit minutes, donc à contre-courant de la circulation de liquide, l'injection de solvant, le prélèvement d'extrait, l'injection de charge et le prélèvement de raffinat.

Selon l'invention, le nombre de lits à considérer n'est donc plus que de six. Huit colonnes (donc deux lits) sont comprises entre l'injection de solvant et le prélèvement d'extrait, quatre colonnes (donc un lit) sont comprises entre le prélèvement d'extrait et l'introduction de charge, huit colonnes (donc deux lits) sont comprises entre l'introduction de charge et le prélèvement de raffinat, enfin les quatre dernières colonnes (un lit) se situent entre le prélèvement de raffinat et l'injection de solvant. On injecte en continu (exprimé aux conditions ambiantes) 10,35 cm$^3$/min de toluène et 7,65 cm$^3$/min de la charge de l'exemple 1. On prélève également en continu 9,35 cm$^3$/min d'extrait et 8,55 cm$^3$/min de raffinat soit environ 5,5 ‰ de pertes). Pendant la première période du cycle qui en compte six, la pompe de recyclage débite (à température ambiante) 35,5 cm$^3$/min, pendant les deux périodes suivantes le débit est de 45,85 cm$^3$/min, pendant la quatrième période le débit de recyclage est 36,5 cm$^3$/min enfin pendant les deux dernières périodes du cycle la pompte débite 44,15 cm$^3$/min. On a donc un débit de recyclage moyen de 42 cm$^3$/min, soit un taux de recyclage moyen de 5,5 exprimé par rapport à la charge. Le paraxylène est obtenu avec une pureté de 87,25% et un taux de récupération de 99,10%. La température est de 150°C. Le tableau suivant donne le bilan en régime stationnaire de l'unité :

|  | Charge | Solvant | Extrait | Raffinat | Pertes |
|---|---|---|---|---|---|
| Débit | 7,65 cm$^3$/min | 10,35cm$^3$/min | 9,35 cm$^3$/min | 8,55 cm$^3$/min | 0,1 cm$^3$/min |
| Toluène | - | 99,9% | 80,58% | 32,21% | |
| Ethylbenzène | 17% | - | 1,23% | 13,79% | |
| Métaxylène | 44% | - | 0,89% | 38,20% | |
| Orthoxylène | 18% | - | 0,36% | 15,63% | |
| P-Xylène | 21% | - | 16,94% | 0,17% | |

**EXEMPLE 4**

L'aspect particulier de l'invention illustré par cet exemple est la production de paraxylène en tant que raffinat avec une mise en oeuvre en co-courant simulé.

L'unité pilote de l'exemple 1 est chargé avec une zéolite de l'exemple 2, tandis que la répartition des colonnes, des lits et les mouvements des vannes sont identiques à celles de l'exemple No. 3.

On injecte en continu (exprimé aux conditions ambiantes) 9,95 cm$^3$/min de toluène et 5,85 cm$^3$/min de charge de l'exemple No.1. On prélève également en continu 11,20 cm$^3$/min d'extrait et 4,53 cm$^3$/min de raffinat ( soit environ 5 ‰ de pertes). Pendant la première période du cycle qui en compte six, la pompe de recyclage débite (à température ambiante) 37,35 cm$^3$/min, pendant les deux périodes suivantes, le débit est de 47,30 cm$^3$/min, pendant la quatrième période le débit de recyclage est de 36,1 cm$^3$/min, enfin pendant les deux dernières périodes du cycle la pompe débite 41,95 cm$^3$/min. On a donc un débit moyen de 42 cm$^3$/min soit un taux de recyclage moyen de 7,18 exprimé par rapport à la charge, le paraxylène est obtenu avec une pureté de 83,36% et un taux de récupération de 97,44%, la température est de 150°C. Le tableau suivant donne le bilan en régime stationnaire de l'unité :

| | Charge | Solvant | Extrait | Raffinat | Pertes |
|---|---|---|---|---|---|
| Débit | 5,85 cm$^3$/min | 9,95 cm$^3$/min | 11,20 cm$^3$/min | 4,53 cm$^3$/min | 0,07 cm$^3$/min |
| Toluène | - | 99,9% | 60,76% | 68,44% | |
| Ethylbenzène | 17% | - | 8,09% | 1,87% | |
| Métaxylène | 44% | - | 22,18% | 1,74% | |
| Orthoxylène | 18% | - | 8,69% | 1,64% | |
| Paraxylène | 21% | - | 0,28% | 26,31% | |

**EXEMPLE 5.**

L'extrait et le raffinat de l'exemple 1 sont repris et distillés en continu dans les conditions suivantes :

| | EXTRAIT | | RAFFINAT | |
|---|---|---|---|---|
| Nombre de plateaux réels | 40 | | 32 | |
| Température de tête | 125°C | | 125°C | |
| Température de fond | 161°C | | 161°C | |
| Taux de reflux | 1,14 | | 1,06 | |
| Composition (% en poids) | | | | |
| Tête | Toluène | 99,48 | Toluène | 98,0 |
| | Ethylbenzène | 0,03 | Ethylbenzène | 0,41 |
| | Paraxylène | 0,48 | Paraxylène | 0,01 |
| | Métaxylène | 0,01 | Métaxylène | 1,14 |
| | orthoxylène | 0,004 | orthoxylène | 0,44 |
| Fond | Toluène | 2,00 | Toluène | 1,53 |
| | Ethylbenzène | 5,07 | Ethylbenzène | 20,35 |
| | Paraxylène | 90,33 | Paraxylène | 0,50 |
| | Métaxylène | 1,89 | Métaxylène | 56,09 |
| | orthoxylène | 0,71 | orthoxylène | 21,53 |

Le solvant réinjecté dans l'unité de chromatographie liquide (constitué par les effluents sortant en tête de colonne) ont la composition suivante :

toluène 98,95%, éthylbenzène 0,21%, métaxylène 0,40%, orthoxylène 0,15%, paraxylène 0,29%, ce qui n'affectera que très peu les compositions d'extrait et de raffinat obtenu dans l'exemple 1 avec du toluène pur à 99,9%. Cela permet par contre d'abaisser très sensiblement les nombres de plateaux ainsi que les taux de reflux et de rebouillage requis par ces deux colonnes par rapport au cas où elles devraient produire du toluène à 99,9%. Le fond de la colonne de raffinat est isomérisé sur un catalyseur bifonctionnel platine sur alumine et mordenite à 390°C sous une pression de 20 bar d'hydrogène et une vitesse spatiale de 3,5 kg par kg et par heure. On obtient un effluent dont la composition pondérale est la suivante :

| légers C1 à benzène inclus | 2,35% |
|---|---|
| toluène | 2,4% |
| naphténiques | 2,2% |
| éthylbenzène | 10,95% |
| paraxylène | 19,72% |
| métaxylène | 42,65% |
| orthoxylène | 19,73% |

ce qui correspond à un taux de conversion des $C_8$ aromatiques de 94,5% et à un taux de conversion de l'éthylbenzène de 43%. Il est à noter qu'il n'est pas possible d'enlever les impuretés naphténiques de l'isomérat. Une concentration stationnaire en ces espèces s'établit dans la boucle, ces produits sont soutirés avec le raffinat dans l'unité de chromatographie liquide.

Le fond de la colonne d'extrait est envoyé dans une unité de cristallisation travaillant à -8°C. On recueille d'une part une liqueur mère contenant environ 38,5% de paraxylène qui est recyclé à l'entrée de l'unité de chromatographie liquide et d'autre part un gâteau de cristaux imbibé de liqueur mère qui est relavé par du toluène provenant de la colonne à distiller de l'extrait (1,15 volume de toluène par volume de gâteaux).

On recueille, après ce lavage, un gâteau de paraxylène dont la teneur en toluène est d'environ 3,1% poids et 1,13 volume de liquide contenant environ 30% de liqueur mère qui est renvoyé vers la colonne à distiller du raffinat. Le paraxylène obtenu est d'une pureté de 99,8%.

La purification finale par cristallisation fonctionnant avec du toluène produit par une des colonnes à distiller de l'unité de chromatographie, l'étape de cristallisation ne requiert par conséquent qu'une colonne à distiller pour la séparation finale du paraxylène et du toluène.

Donc la combinaison des étapes du procédé selon l'invention permet de minimiser la taille des colonnes à distiller et donc leur consommation énergétique. Elle permet aussi de réduire la consommation en solvant.

De manière similaire le raffinat et l'extrait produits dans les exemples 2, 3 et 4 sont distillés en continu. Le raffinat sensiblement exempt de solvant est isomérisé dans les conditions définies ci-dessus, tandis que l'extrait sensiblement exempt de solvant est envoyé dans une unité de cristallisation décrite plus haut, avec laquelle on produit du paraxylène à 99,8% et une liqueur mère qui est renvoyée vers l'étape de chromatographie en lit mobile simulé.

## Revendications

1. Procédé de séparation et de récupération de paraxylène contenu dans une charge d'hydrocarbures comprenant essentiellement des hydrocarbures aromatiques en $C_8$, le procédé comportant une étape d'adsorption de la charge délivrant une première fraction et une seconde fraction, une étape d'isomérisation de la première fraction et une étape de cristallisation de la seconde fraction, le procédé étant caractérisé en ce qu'il comprend les étapes suivantes :

a) on met en contact en continu dans au moins une zone d'adsorption à lit mobile simulé, ladite charge contenant du métaxylène, du paraxylène, de l'éthylbenzène et éventuellement de l'orthoxylène avec un lit d'adsorbant zéolitique en présence d'un solvant de désorption approprié dont le débit par rapport à celui de la charge est de 1,2 à 2,5 dans des conditions d'adsorption telles qu'on obtient la première fraction contenant du solvant, du métaxylène, de l'éthylbenzène et éventuellement de l'orthoxylène et la seconde fraction contenant du solvant et essentiellement du paraxylène avec une pureté comprise entre 75 et 98% et avec une productivité améliorée ;

b) on distille la première fraction pour séparer le solvant d'une part et le mélange métaxylène, éthylbenzène et éventuellement orthoxylène d'autre part ;

c) on isomérise ledit mélange dans des conditions appropriées en présence d'hydrogène dans une zone d'isomérisation et l'on récupère un isomérat que l'on recycle vers l'étape a) ;

d) on distille la deuxième fraction et l'on récupère le solvant d'une part et le paraxylène avec une pureté de 75 à 98% d'autre part ;

e) on procède à au moins une cristallisation du paraxylène de l'étape d) dans une zone de cristallisation à une température comprise entre + 10°C et - 25°C et l'on obtient d'une part une liqueur mère que l'on recycle vers l'étape a) et d'autre part des cristaux de paraxylène imbibés de liqueur mère ;

f) on lave avec un solvant de lavage approprié les cristaux de paraxylène dans une zone de lavage et l'on récupère les cristaux de paraxylène à un très grand degré de pureté.

2. Procédé selon la revendication 1 dans lequel le lit mobile simulé est à contre-courant.

3. Procédé selon la revendication 1 dans lequel le lit mobile simulé est à co-courant.

4. Procédé selon l'une des revendications 1 à 3 dans lequel la première fraction est un raffinat et la deuxième fraction un extrait.

5. Procédé selon l'une des revendications 1 à 3 dans lequel la première fraction et un extrait et la deuxième fraction un raffinat.

6. Procédé selon l'une des revendications 1 à 5 dans lequel ladite charge contient de l'orthoxylène qui est séparé par distillation avant l'étape a).

7. Procédé selon l'une des revendications 1 à 6 dans lequel la charge est une coupe hydrocarbures de point d'ébullition compris entre 136°C et 145°C.

8. Procédé selon l'une des revendications 1 à 7 dans lequel la seconde fraction de l'étape a) contient du paraxylène dont la pureté est comprise entre 85 et 90%.

9. Procédé selon l'une des revendications 1 à 8 dans lequel les conditions d'adsorption dans l'unité d'adsorption à lit mobile simulé sont les suivantes :
température : 140 - 185°C,
taux de solvant : 1,35 - 1,7
vitesse linéaire moyenne rapportée au réacteur vide : 0,4 - 1,2 cm.s$^{-1}$
nombre de lits : 6 - 24
nombre de zones : au moins 4.

10. Procédé selon l'une des revendications 1 à 9 dans lequel la cristallisation du paraxylène lors de l'étape e) est effectuée à une température de + 5 à - 15°C.

11. Procédé selon l'une des revendications 1 à 10 dans lequel le solvant de désorption et le solvant de lavage sont un seul et même solvant.

12. Procédé selon l'une des revendications 1 à 10 dans lequel le solvant de désorption est un solvant de point d'ébullition supérieur à celui de la charge tel que le paradiéthylbenzène et dans lequel le solvant de lavage est un solvant de point d'ébullition inférieur à celui de la charge tel que le toluène.

13. Procédé selon la revendication 11 dans lequel le solvant récupéré lors de l'étape b) est recyclé dans la zone d'adsorption et/ou dans la zone de lavage, le solvant impur résultant du lavage est recyclé à l'étape b) et/ou à l'étape d) de distillation et dans lequel le solvant résultant de l'étape d) de distillation est recyclé vers la zone d'adsorption et/ou vers la zone de lavage.

14. Procédé selon la revendication 12 dans lequel le solvant récupéré lors de l'étape b) et d) de distillation est recyclé dans la zone d'adsorption et dans lequel le solvant impur résultant du lavage est soumis à une distillation séparée adaptée à délivrer du solvant pur recyclé vers l'étape de lavage dans la zone de cristallisation et un mélange des constituants de la charge recyclé vers l'étape a) d'adsorption.

15. Unité de séparation et de récupération de paraxylène contenu dans une charge d'hydrocarbures comprenant essentiellement des hydrocarbures aromatiques en C$_8$ caractérisée en ce qu'elle comporte en combinaison :
a) une unité (8) d'adsorption dite à lit mobile simulé comprenant une pluralité de colonnes (6, 7) remplies d'un adsorbant zéolitique, un moyen d'alimentation (4) en une charge, un moyen d'alimentation (11) en un solvant de désorption recyclé, un moyen d'évacuation (10) d'une première fraction et un moyen d'évacuation (9) d'une seconde fraction contenant du paraxylène, ladite unité d'adsorption étant adaptée à délivrer la seconde fraction à une pureté appropriée et avec une productivité améliorée ;

b) une première unité de distillation (12) connectée audit moyen d'évacuation de la première fraction comprenant une sortie (14) adaptée à délivrer le solvant qui est recyclé au moins en partie par ledit moyen d'alimentation (11) vers l'unité d'adsorption et une deuxième sortie (15) adaptée à délivrer un raffinat débarrassé du solvant ;

c) une unité d'isomérisation (21) ayant une entrée connectée à la sortie (15) de l'unité de distillation (12) et une sortie délivrant un isomérat reliée à une troisième unité de distillation (23) définie ci-dessous ;

d) une troisième unité (23) de distillation adaptée à délivrer par une sortie un isomérat distillé qui est recyclé par les moyens appropriés (2) vers l'unité d'adsorption et par une autre sortie des produits légers produits au cours de l'isomérisation ;

e) une deuxième unité de distillation (16) connectée audit moyen d'évacuation (9) de la deuxième fraction contenant le paraxylène comprenant une sortie (17) adaptée à délivrer le solvant de désorption qui est recyclé au moins en partie par le moyen de recyclage (11) vers l'unité d'adsorption et une deuxième sortie (19) adaptée à délivrer du paraxylène de pureté comprise habituellement entre 75 et 98% ;

f) au moins une unité de cristallisation du paraxylène de l'étape e) connectée à la sortie (19) adaptée à fonctionner à une température de -25°C à + 10°C, ladite unité de cristallisation comprenant en outre une unité de lavage des cristaux obtenus qui comporte une alimentation (18) en un solvant de lavage approprié, une première sortie délivrant une solution mère qui est recyclée par des moyens de recyclage (3) vers l'unité d'adsorption et une seconde sortie (25) de récupération des cristaux purs ;

l'unité de séparation et de récupération du paraxylène étant caractérisée en ce qu'une ligne (18) de solvant de lavage relie la sortie (14) de la première unité de distillation et/ou la première sortie (17) de la deuxième unité de distillation (12), à l'unité de lavage des cristaux et en ce que ladite unité de lavage est connectée par une ligne (13) de récupération de solvant impur à la première unité de distillation adaptée à séparer du solvant sensiblement pur.

**16.** Unité selon la revendication 15 dans laquelle les moyens de recyclage (3) sont connectés au moyen d'alimentation en la charge des colonnes (6).

**17.** Unité selon les revendications 15 et 16 dans laquelle le lit mobile simulé est un lit à contre-courant simulé.

**18.** Unité selon les revendications 15 et 16 dans laquelle le lit mobile simulé est un lit à co-courant simulé.

**Claims**

1. Process for the separation and recovery of p-xylene contained in a hydrocarbon charge essentially constituted by $C_8$ aromatic hydrocarbons,

the process comprising a stage of adsorbing the charge supplying a first fraction and a second fraction, a stage of isomerizing the first fraction ,and a stage of crystallizing the second fraction, the process being characterized in that it comprises the following stages:

a) continuously contacting in at least one simulated moving bed adsorption zone, the said charge containing m-xylene, p-xylene, ethyl benzene and optionally o-xylene with a zeolitic adsorbent bed in the presence of an appropriate desorption solvent, whose flow rate compared with that of the charge is 1.2 to 2.5 under adsorption conditions such that the first fraction is obtained containing the solvent, m-xylene, ethyl benzene and optionally o-xylene and the second fraction containing the solvent and essentially p-xylene with a purity between 75 and 98% and with an improved productivity;

b) the first fraction is distilled to separate the solvent on the one hand and the mixture of m-xylene, ethyl benzene and optionally o-xylene on the other;

c) the mixture is isomerized under appropriate conditions in the presence of hydrogen in an isomerization zone and an isomerate is recovered which is recycled to stage a)

d) the second fraction is distilled and the solvent recovered on the one hand and the p-xylene with a purity of 75 to 98% on the other;

e) At least one crystallization takes place of the p-xylene of stage d) in a crystallization zone at a temperature between + 10°C and -25°C and on the one hand a mother liquor is obtained, which is recycled to stage a), and on the other mother liquor-impregnated p-xylene crystals;

f) using an appropriate washing solvent washing takes place of the p-xylene crystals in a washing zone and the p-xylene crystals with a very high purity level are recovered.

2. Process according to claim 1, wherein a simulated countercurrent moving bed is used.

3. Process according to claim 1, wherein a simulated cocurrent moving bed is used.

4. Process according to any one of the claims 1 to 3, wherein the first fraction is a raffinate and the second fraction an extract.

5. Process according to any one of the claims 1 to 3, wherein the first fraction is an extract and the second fraction a raffinate.

6. Process according to any one of the claims 1 to 5, wherein the charge contains o-xylene, which is separated by distillation prior to stage a).

7. Process according to any one of the claims 1 to 6, wherein the charge is a hydrocarbon fraction with a boiling point between 136 and 145°C.

8. Process according to any one of the claims 1 to 7, wherein the second fraction of stage a) contains p-xylene with a purity between 85 and 90%.

9. Process according to any one of the claims 1 to 8, wherein the adsorption conditions in the simulated moving bed adsorption unit are temperature 140 to 185°C, solvent rate 1.35 to 1.7, average linear velocity based on the empty reactor 0.4 to 1.2 cm.s$^{-1}$, number of beds 6 to 24 and at least 4 zones.

10. Process according to any me of the claims 1 to 9, wherein the crystallization of the p-xylene during stage e) is performed at a temperature from +5 to -15°C.

11. Process according to any one of the claims 1 to 10, wherein the desorption solvent and the washing solvent are one and the same solvent.

12. Process according to any one of the claims 1 to 10, wherein the desorption solvent is a solvent having a boiling point higher than that of the charge, such as p diethyl benzene and in which the washing solvent is a solvent having a boiling point lower than that of the charge, such as toluene.

13. Process according to claim 11, wherein the solvent recovered during stage b) is recycled to the adsorption zone and/or washing zone, the impure solvent resulting from the washing is recycled to stage b) and/or to the distillation stage d) and wherein the solvent resulting from the distillation stage d) is recycled to the adsorption zone and/or to the washing zone.

14. Process according to claim 12, wherein the solvent recovered during stage b) and distillation stage d) is recycled to the adsorption zone and wherein the impure solvent resulting from the washing undergoes a separate distillation suitable for supplying pure recycled solvent to the washing stage in the crystallization zone and a mixture of the constituents of the recycled charge to the adsorption stage.

15. Unit for the separation and recovery of p-xylene contained in a hydrocarbon charge essentially constituted by C$_8$ aromatic hydrocarbons, characterized in that it comprises in combination:

a) a simulated moving bed adsorption unit (8) having a plurality of columns (6, 7) filled with a zeolitic adsorbent, a means (4) for supplying a charge, a means (11) for supplying a recycled desorption solvent, a means (10) for the discharge of the first fraction and a means (9) for the discharge of a second fraction containing p-xylene, said adsorbtion unit being able to supply said fraction with an appropriate purity ant with an improved productivity;

b) a first distillation unit (12) connected to said means for the discharge of the first fraction and having an outlet (14) able to supply the solvent recycled at least partly by said supply means (11) to the adsorbtion unit and a second outlet (15) able to supply a solvent-free raffinate ;

c) an isomerization unit (21) having an inlet connected to the outlet (15) of the distillation unit (12) and an outlet supplying an isomerate connected to a third distillation unit (23) defined hereinafter;

d) a third distillation unit (23) able to supply by one outlet a distilled isomerate, which is recycled by appropriate means (2) to the adsorption unit ant by another outlet light products produced during the isomerization;

e) a second distillation unit (16) connected to said discharge means (9) for the second fraction containing the p-xylene and having an outlet (17) able to supply the desorption solvent, which is at least partly recycled by the recycling means (11) to the adsorption unit and a second outlet (19) able to supply p-xylene with a purity normally between 75 and 98%;

f) at least one unit for the crystallization of the p-xylene of stage e) connected to the outlet (19) and able to operate at a temperature of -25 to +10°C, said crystalization unit also comprising a unit for washing crystals obtained ant which has a supply means (18) for an appropriate washing solvent, a first outlet supplying a mother solution recycled by the recycling means (3) to the adsorption unit and a second pure crystal recovery outlet (25); the p-xylene separation and recovery unit being characterized in that a washing solvent line (18) connects the outlet (14) of the first distillation unit and/or the first outlet (17) of the second distillation unit (12) to the crystal washing unit and in that the washing unit is connected by an impure solvent recovery line (13) to the first distillation unit able to separate it from the substantially pure solvent.

16. Unit according to claim 15, wherein the recycling means (3) are connected to the means for supplying charge to the columns (6).

17. Unit according to claims 15 and 16, wherein the simulated moving bed is a simulated countercurrent bed.

18. Unit according to claims 15 and 16, wherein the simulated moving bed is a simulated cocurrent bed.

**Patentansprüche**

1. Verfahren zur Trennung und zur Wiedergewinnung von Paraxylol, das in einer Charge aus Kohlenwasserstoffen enthalten ist, welche im wesentlichen aromatische $C_8$-Kohlenwasserstoffe umfassen, wobei das Verfahren einen Schritt zur Adsorption der Charge, der eine erste Fraktion und eine zweite Fraktion liefert, einen Schritt zur Isomerisierung der ersten Fraktion und einen Schritt zur Kristallisation der zweiten Fraktion umfaßt, wobei das verfahren dadurch gekennzeichnet ist, daß es die folgenden Schritte umfaßt:

a) man bringt die Charge, die Metaxylol, Paraxylol, Ethylbenzol und gegebenenfalls Orthoxylol enthält, in wenigstens einer Adsorptionszone mit simuliertem Fließbett kontinuierlich in Kontakt mit einem Bett aus zeolithischem Adsorbens in Anwesenheit eines geeigneten Desorptionsmittels, dessen Menge in bezug auf diejenige der Charge 1,2 bis 2,5 ist, unter Adsorptionsbedingungen, derart, daß man die erste Fraktion, welche Lösungsmittel, Metaxylol, Ethylbenzol und gegebenenfalls Orthoxylol enthält, und die zweite Fraktion, welche Lösungsmittel und im wesentlichen Paraxylol mit einer Reinheit einschließlich zwischen 75 und 98 % und mit einer verbesserten Produktivität enthält, erhält;

b) man destilliert die erste Fraktion um das Lösungsmittel einerseits und die Metaxylol-, Ethylbenzol- und gegebenenfalls Orthoxylol-Mischung andererseits zu trennen;

c) man isomerisiert die Mischung unter geeigneten Bedingungen in Anwesenheit von Wasserstoff in einer Isomerisierungszone und man gewinnt ein Isomer wieder, das man zum Schritt a) zurückführt;

d) man destilliert die zweite Fraktion und man gewinnt das Lösungsmittel einerseits und das Paraxylol mit einer Reinheit von 75 bis 98 % andererseits wieder;

e) man führt wenigstens eine Kristallisation des Paraxylols aus dem Schritt d) in einer Kristallisationszone bei einer Temperatur von einschließlich zwischen +10°C und -25°C durch und man erhält zum einen eine Stammlösung, die man dem Schritt a) zurückführt und zum anderen Paraxylolkristalle, welche von der Stammlösung aufgenommen sind;

f) man wäscht mit einem geeigneten Waschmittel die Paraxylolkristalle in einer Waschzone und man gewinnt die Paraxylolkristalle mit einem sehr hohen Reinheitsgrad zurück.

2. Verfahren nach Anspruch 1, bei welchem das simulierte Fließbett im Gegenstrom betrieben wird.

**3.** Verfahren nach Anspruch 1, bei welchem das simulierte Fließbett im Gleichstrom betrieben wird.

**4.** Verfahren nach einem der Ansprüche 1 bis 3, bei welchem die erste Fraktion ein Raffinat und die zweite Fraktion ein Extrakt ist.

**5.** Verfahren nach einem der Ansprüche 1 bis 3, bei welchem die erste Fraktion ein Extrakt und die zweite Fraktion ein Raffinat ist.

**6.** Verfahren nach einem der Ansprüche 1 bis 5, bei welchem die Charge, die das Orthoxylol enthält, durch Destillation vor dem Schritt a) getrennt wird.

**7.** Verfahren nach einem der Ansprüche 1 bis 6, bei welchem die Charge ein Kohlenwasserstoffschnitt mit einem Siedepunkt einschließlich zwischen 136 °C und 145 °C ist.

**8.** Verfahren nach einem der Ansprüche 1 bis 7, bei welchem die zweite Fraktion aus dem Schritt a) Paraxylol enthält, dessen Reinheit einschließlich zwischen 85 und 90 % beträgt.

**9.** Verfahren nach einem der Ansprüche 1 bis 8, bei welchem die Adsorptionsbedingungen in der Einheit zur Adsorption mit simuliertem Fließbett die folgenden sind:
Temperatur: 140 - 185 °C,
Lösungsmittelfaktor: 1,35 - 1,7,
mittlere Lineargeschwindigkeit,
bezogen auf den leeren Reaktor: 0,4 - 1,2 cm.s$^{-1}$,
Anzahl der Betten: 6 - 24,
Anzahl der Zonen: mindestens 4.

**10.** Verfahren nach einem der Ansprüche 1 bis 9, bei welchem die Kristallisation des Paraxylols während des Schrittes e) bei einer Temperatur von +5 bis -15 °C durchgeführt wird.

**11.** Verfahren nach einem der Ansprüche 1 bis 10, bei welchem das Desorptionsmittel und das Waschmittel ein einziges und gleiches Lösungsmittel sind.

**12.** Verfahren nach einem der Ansprüche 1 bis 10, bei welchem das Desorptionsmittel ein Lösungsmittel mit einem Siedepunkt größer als derjenige der Charge, wie das Paradiethylbenzol, ist und bei welchem das Waschmittel ein Lösungmittel mit einem Siedepunkt kleiner als derjenige der Charge, wie das Toluol, ist.

**13.** Verfahren nach Anspruch 11, bei welchem das Lösungsmittel, das während des Schrittes b) wiedergewonnen wird, in die Adsorptionszone und/oder in die Waschzone zurückgeführt wird, wobei das unreine Lösungsmittel, das aus dem Waschen resultiert, beim Destillationsschritt b) und/oder beim Destillationsschritt d) zurückgeführt wird, und bei welchem das Lösungsmittel, das aus dem Destillationsschritt d) resultiert, in die Adsorptionszone und/oder in die Waschzone zurückgeführt wird.

**14.** Verfahren nach Anspruch 12, bei welchem das Lösungsmittel, das während des Destillationsschrittes b) und des Destillationsschrittes d) zurückgewonnen wird, in die Adsorptionszone zurückgeführt wird und bei welchem das unreine Lösungsmittel, das aus dem Waschen resultiert, einer getrennten Destillation unterworfen wird, die geeignet ist, reines Lösungsmittel, welches zum Waschschritt in die Kristallisationszone zurückgeführt wird, und eine Mischung von Bestandteilen der Charge, die zum Adsorptionsschritt a) zurückgeführt wird, zu liefern.

**15.** Einheit zur Trennung und zur Wiedergewinnung von Paraxylol, das in einer Charge aus Kohlenwasserstoffen enthalten ist, welche im wesentlichen aromatische $C_8$-Kohlenwasserstoffe umfaßt, dadurch gekennzeichnet, daß sie in Kombination umfaßt:
a) eine Einheit (8) zur Adsorption mit einem simulierten Fließbett, die eine Vielzahl von Kolonnen (6, 7), welche mit einem zeolithischen Adsorbens gefüllt sind, eine Einrichtung (4) zur Zufuhr einer Charge, eine Einrichtung (11) zur Zufuhr eines zurückgeführten Desorptionsmittels, eine Einrichtung (10) zum Abzug einer ersten Fraktion und eine Einrichtung (9) zum Abzug einer zweiten Fraktion, die Paraxylol enthält, umfaßt, wobei die Einheit zur Adsorption geeignet ist, die zweite Fraktion mit einer geeigneten Reinheit und mit einer verbesserten Produktivität zu liefern;

b) eine erste Einheit (12) zur Destillation, die mit der Einrichtung zum Abzug der ersten Fraktion verbunden ist, welche einen ersten Auslaß (14), der geeignet ist, das Lösungsmittel, welches zurückgeführt wird, wenigstens teilweise über die Einrichtung (11) zur Zufuhr zu der Einheit zur Adsorption zu liefern, und einen zweiten Auslaß (15), der geeignet ist, ein abgezogenes Raffinat des Lösungsmittels zu liefern, umfaßt;

c) eine Einheit (21) zur Isomerisierung, die einen Einlaß, welcher mit dem Auslaß (15) der Einheit (12) zur Destillation verbunden ist, und einen ein Isomer liefernden Auslaß, welcher mit einer unten definierten, dritten Einheit (23) zur Destillation verbunden ist, aufweist;

d) eine dritte Einheit (23) zur Destillation, die geeignet ist, über einen Auslaß ein destilliertes Isomer zu liefern, welches durch die geeigneten Einrichtungen (2) zu der Einheit zur Adsorption zurückgeführt wird, und über einen anderen Auslaß leichte Produkte, welche während der Isomerisierung hergestellt werden, zu liefern;

e) eine zweite Einheit (16) zur Destillation, die mit der Einrichtung (9) zum Abzug der zweiten Fraktion, welche das Paraxylol enthält, verbunden ist, einen Auslaß (17), welcher geeignet ist, das Desorptionsmittel, das wenigstens teilweise durch die Einrichtung (11) zur Zurückführung zu der Einheit zur Adsorption zürückgeführt wird, zu liefern, sowie einen zweiten Auslaß (19), welcher geeignet ist, Paraxylol von einer Reinheit gewöhnlicherweise einschließlich zwischen 75 und 98 % zu liefern, umfaßt;

f) wenigstens eine Einheit, zur Kristallistion des Paraxylols aus dem Schritt e), die mit dem Ausgang (19) verbunden und geeignet ist, bei einer Temperatur von -25°C bis +10°C zu arbeiten, wobei die Einheit zur Kristallisation weiterhin eine Einheit zum Waschen von erhaltenen Kristallen, die eine Zufuhr (18) für ein geeignetes Waschmittel umfaßt, einen ersten Auslaß, der ein Stammlösungsmittel liefert, welches durch die Einrichtungen (3) zur Zurückführung zu der Einheit zur Adsorption zurückgeführt wird, und einen zweiten Auslaß (25) zur Wiedergewinnung reiner Kristalle umfaßt;

wobei die Einheit zur Trennung und zur Wiedergewinnung von Paraxylol dadurch gekennzeichnet ist, daß eine Leitung (18) für Waschmittel den Auslaß (14) der ersten Einheit zur Destillation und/oder den ersten Auslaß (17) der zweiten Einheit (12) zur Destillation mit der Einheit zum Waschen der Kristalle verbindet und daß die Einheit zum waschen über eine Leitung (13) zur Wiedergewinnung von unreinem Lösungsmittel mit der ersten Einheit zur Destillation, die geeignet ist, im wesentlichen reines Lösungsmittel abzutrennen, verbunden ist.

16. Einheit gemäß Anspruch 15, bei welcher die Einrichtungen (3) zur Zurückführung mit einer Einrichtung zur Zufuhr zur Charge der Kolonnen (6) verbunden ist.

17. Einheit gemäß den Ansprüchen 15 und 16, bei welcher das simulierte Fließbett ein simuliertes Bett im Gegenstrom ist.

18. Einheit gemäß den Ansprüchen 15 und 16, bei welcher das simulierte Fließbett ein simuliertes Bett im Gleichstrom ist.